(19) Europäisches Patentamt
European Patent Office
Office européen des brevets

(11) **EP 3 378 948 B1**

(12) **EUROPEAN PATENT SPECIFICATION**

(45) Date of publication and mention
of the grant of the patent:
**04.11.2020 Bulletin 2020/45**

(51) Int Cl.:
**C12Q 1/68** *(2018.01)*    **C12N 15/09** *(2006.01)*

(21) Application number: **16866395.3**

(86) International application number:
**PCT/JP2016/084136**

(22) Date of filing: **17.11.2016**

(87) International publication number:
**WO 2017/086394 (26.05.2017 Gazette 2017/21)**

(54) **METHOD FOR QUANTIFYING TARGET NUCLEIC ACID AND KIT THEREFOR**

VERFAHREN ZUR QUANTIFIZIERUNG VON ZIELNUKLEINSÄURE UND KIT DAFÜR

PROCÉDÉ POUR QUANTIFIER UN ACIDE NUCLÉIQUE CIBLE ET KIT ASSOCIÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.11.2015 JP 2015225671**

(43) Date of publication of application:
**26.09.2018 Bulletin 2018/39**

(73) Proprietor: **Japan Agency for Marine-Earth
Science and
Technology
Yokosuka-shi, Kanagawa 237-0061 (JP)**

(72) Inventors:
• **HOSHINO, Tatsuhiko
Yokosuka-shi
Kanagawa 237-0061 (JP)**
• **INAGAKI, Fumio
Yokosuka-shi
Kanagawa 237-0061 (JP)**

(74) Representative: **Schulz Junghans
Patentanwälte PartGmbB
Großbeerenstraße 71
10963 Berlin (DE)**

(56) References cited:
WO-A1-2013/128281    WO-A2-2007/129000
WO-A2-2012/051504    JP-A- 2006 525 809
JP-A- 2007 295 896    JP-A- 2012 531 202
JP-A- 2013 515 458    US-A1- 2015 197 786

• TATSUHIKO HOSHINO ET AL: "Molecular
quantification of environmental DNA using
microfluidics and digital PCR", SYSTEMATIC
AND APPLIED MICROBIOLOGY., vol. 35, no. 6, 1
September 2012 (2012-09-01), pages 390-395,
XP055564727, AMSTERDAM, NL ISSN:
0723-2020, DOI: 10.1016/j.syapm.2012.06.006

**Description**

Technical Field

The present invention relates to a method for quantifying a target nucleic acid containing a sequence of interest flanked by conserved regions.

Background Art

Development of next-generation sequencing technologies has enabled massively parallel sequencing of DNA or RNA. For sequencing, a target gene of interest is amplified by PCR to an extent that allows the detection of the target.
The relative abundance (i.e., ratio) of a target sequence in a sequence library generated by the next-generation sequencing technologies can be ideally quantitative. In practice, however, it is difficult to estimate the copy number of a target sequence in a sample from the ratio in a sequence library. This is because the efficiency of PCR amplification may vary depending on the sequence, GC content and secondary structure of target sequence. For this reason, additional quantitative assays such as real-time PCR should be performed to accurately quantify target sequences.
A method that addresses the above-described problems arising from the heterogeneity of amplification efficiency of PCR among different sequences and that enables simultaneous sequencing and quantification of the target nucleic acid is described in Patent Document 1 and Non-Patent Document 1. In this method, a target sequence ligated to an adapter sequence consisting of a consensus sequence and a variable sequence at the terminal is used as a template and PCR is performed using a primer having a sequence complementary to the consensus sequence. Subsequently, the sequence of the resulting amplicon is determined to calculate the copy number of the target nucleic acid based on the type of the variable sequence and its number.
In Patent Document 2 and 4, the target sequence is tagged for counting using a counter sequence.
In Patent Document 3, quantification of target sequences is performed via amplification of a plurality of aliquots containing the target sequence.

Prior Art Documents

Patent Documents

Patent Document 1: US 2011/0160078 A
Patent Document 2: WO 2013/128281 A1
Patent Document 3: WO 2007/129000 A2
Patent Document 4: US 2015/197786 A1

Non-Patent Documents

Non-Patent Document 1: Glenn K. Fu et al., PNAS, May 31, 2011, vol.108, no.22, pp.9026-9031

Disclosure of the Invention

Problems to Be Solved by the Invention

However, the method described in Patent Document 1 and Non-Patent Document 1, in which a specific sequence is ligated to the terminal of a target nucleic acid, requires treatment with a restriction enzyme to form a protruding end at the terminal of the target nucleic acid for the ligation. The formation of protruding ends may be difficult or impossible, depending on the sequence of the target nucleic acid. Also, the efficiency of cleavage by the restriction enzyme may affect the efficiency of the formation of protruding ends.
It is therefore an objective of the present invention to provide a method that enables sequencing and quantification of a target nucleic acid without requiring a ligation reaction and that addresses the problems arising from the amplification efficiency of PCR.

Means for Solving the Problems

As a result of extensive studies to find solutions to the above-identified problems, the present inventors successfully conceived of a method comprising (1) carrying out a single primer extension reaction using primers containing a sequence specific to the sequence of a target nucleic acid, and random sequences and a known sequence introduced upstream of the specific sequence so as to obtain a double-stranded nucleic acid having the random sequences and the sequence of the target nucleic acid; (2) performing PCR using the resulting double-stranded nucleic acid as a template along with a primer specific to the known sequence and a primer having a part of the sequence of the target nucleic acid so as to obtain an amplicon of the double-stranded nucleic acid containing the random sequences and the sequence of the target nucleic acid; and (3) sequencing the resulting amplicon of the double-stranded nucleic acid to determine the sequence of the target nucleic acid and the random sequences. Furthermore, the present inventors have discovered that the present method can enable quantification of the copy number of target nucleic acid based on the diversity of the random sequences while enabling sequencing of target nucleic acid.

The present inventors have also found that the present method is highly accurate as compared to conventional methods that estimate the copy numbers of target nucleic acids in a sample from the relative abundance of target nucleic acids in a sequence library and also found that the present method allows accurate quantification of the copy numbers of different target nucleic acids not only in mock samples prepared in such a manner that it contains two or more target nucleic acids, but also in environmental samples. These successful examples and findings have ultimately led to the completion of the present invention.

According to one aspect of the present invention, there is provided a method for quantifying a target nucleic acid, comprising the following steps (1) to (4):

(1) carrying out an annealing reaction, a nucleic acid extension reaction, and a nuclease reaction by using as a template a single-stranded nucleic acid of target nucleic acid or a single-stranded nucleic acid obtained from a double-stranded nucleic acid of target nucleic acid that contains in direction from 3' end toward 5' end a first sequence, a sequence of interest and a second sequence, along with a number of first primers that each contain in direction from 5' end toward 3' end a specific sequence, a random sequence and a sequence complementary to the first sequence and that each have a different sequence corresponding to the number of different sequences of the random sequence so as to obtain a first double-stranded nucleic acid with one strand containing in direction from 5' end toward 3' end the specific sequence, the random sequence, the sequence complementary to the first sequence, a sequence complementary to the sequence of interest, and a sequence complementary to the second sequence;

(2) carrying out a PCR using the first double-stranded nucleic acid obtained from the step (1) as a template along with a second primer containing the second sequence and a third primer containing the specific sequence so as to obtain a second double-stranded nucleic acid;

(3) determining the sequence of the second double-stranded nucleic acid obtained from the step (2); and

(4) measuring the number of combinations of the sequence of interest and the random sequence based on the sequence determined in the step (3) and quantifying the target nucleic acid by the following equation (1):

$$N = \ln\left(\frac{C-H}{C}\right) \times (-C)$$

(Equation (1))

wherein N represents the number of target nucleic acids; C represents the number of different sequences of the random sequence; and H represents the measured number of combinations of the sequence of interest and the random sequence.

Also disclosed is a method for amplifying a double-stranded nucleic acid containing a sequence of interest of target nucleic acid and a random sequence, the method comprising the following steps (1) and (2):

(1) carrying out an annealing reaction, a nucleic acid extension reaction, and a nuclease reaction by using as a template a single-stranded nucleic acid of target nucleic acid or a single-stranded nucleic acid obtained from a double-stranded nucleic acid of target nucleic acid that contains in direction from 3' end toward 5' end a first sequence, a sequence of interest and a second sequence, along with a number of first primers that each contain in direction from 5' end toward 3' end a specific sequence, a random sequence and a sequence complementary to the first sequence and that each have a different sequence corresponding to the number of different sequences of the random sequence so as to obtain a first double-stranded nucleic acid with one strand containing in direction from 5' end toward 3' end the specific sequence, the random sequence, the sequence complementary to the first sequence,

a sequence complementary to the sequence of interest and a sequence complementary to the second sequence; and (2) carrying out a PCR using the first double-stranded nucleic acid obtained from the step (1) as a template along with a second primer containing the second sequence and a third primer containing the specific sequence so as to obtain a second double-stranded nucleic acid.

In one embodiment of the method of the present invention, the target nucleic acid is preferably a nucleic acid derived from one or two or more organisms.

In one embodiment of the method of the present invention, the target nucleic acid is preferably a nucleic acid containing a part or all of rRNA gene.

In one embodiment of the method of the present invention, the sequence of interest is preferably a variable region of rRNA gene and the first sequence and the second sequence are downstream and upstream conserved regions of the variable region, respectively.

In one embodiment of the method of the present invention, the target nucleic acid is preferably a nucleic acid in a sample selected from the group consisting of water, soil, air, body tissue, food products, pharmaceutical products and cosmetic products.

In one embodiment of the method of the present invention, the number of the target nucleic acids is preferably any number between 100 and 1,000,000.

In one embodiment of the method of the present invention, the random sequence is preferably a random sequence having any number of bases between 4 and 20 or the number of different sequences of the random sequence is any number between $10^2$ and $10^{15}$.

In one embodiment of the method of the present invention, the specific sequence may preferably have any number of bases between 10 and 50 and comprise a sequence non-complementary to the sequence of the target nucleic acid.

Also disclosed is a kit for quantifying a target nucleic acid that is either a single-stranded nucleic acid containing in direction from 3' end toward 5' end a first sequence, a sequence of interest and a second sequence or a double-stranded nucleic acid containing the single strand, the kit comprising:

a number of first primers that each contain in direction from 5' end toward 3' end a specific sequence, a random sequence and a sequence complementary to the first sequence and that each have a different sequence corresponding to the number of different sequences of the random sequence;
a nuclease;
a second primer containing the second sequence; and
a third primer containing the specific sequence.

In the kit, the sequence of interest is preferably a variable region of rRNA gene and the first sequence and the second sequence are downstream and upstream conserved regions of the variable region, respectively.

Advantages of the Invention

According to the method of one embodiment of the present invention, problems resulting from the heterogeneous amplification efficiency of PCR can be eliminated and accurate quantification of the copy number of target nucleic acids as well as sequencing of target nucleic acids can be simultaneously achieved. The kit can be used to perform the method of one embodiment of the present invention.

Brief Description of the Drawings

FIG. 1 is a schematic diagram showing a method for quantifying a target nucleic acid in accordance with one embodiment of the present invention.
FIG. 2 is an illustrative diagram showing a method used in the experiment as described in Example 1.
FIG. 3 is a graph showing the linearity of the quantified values as described in Example 2.
FIG. 4 is a graph comparing the results of quantification in a complex system as described in Example 2.

Modes for Carrying Out the Invention

One specific embodiment of the present invention is a method for quantifying a target nucleic acid, the method comprising at least the following steps (1) to (4):

(1) carrying out an annealing reaction, a nucleic acid extension reaction, and a nuclease reaction by using as a template a single-stranded nucleic acid of target nucleic acid or a single-stranded nucleic acid obtained from a double-stranded nucleic acid of target nucleic acid that contains in direction from 3' end toward 5' end a first sequence, a sequence of interest and a second sequence, along with a number of first primers that each contain in direction from 5' end toward 3' end a specific sequence, a random sequence and a sequence complementary to the first sequence and that each have a different sequence corresponding to the number of different sequences of the random sequence so as to obtain a first double-stranded nucleic acid with one strand containing in direction from 5' end toward 3' end: the specific sequence, the random sequence, the sequence complementary to the first sequence, a sequence complementary to the sequence of interest, and a sequence complementary to the second sequence;

(2) carrying out a PCR using the first double-stranded nucleic acid obtained from the step (1) as a template along with a second primer containing the second sequence and a third primer containing the specific sequence so as to obtain a second double-stranded nucleic acid.

(3) determining the sequence of the second double-stranded nucleic acid obtained from the step (2); and

(4) measuring the number of combinations of the sequence of interest and the random sequence based on the sequence determined in the step (3) and quantifying the target nucleic acid by the following equation (1):

$$ N \;=\; ln\left(\frac{C-H}{C}\right) \times (-C) $$

$$\text{(Equation (1))}$$

wherein N represents the number of target nucleic acids; C represents the number of different sequences of the random sequence; and H represents the measured number of combinations of the sequence of interest and the random sequence.

The quantification method of one embodiment of the present invention enables comprehensive sequencing of target nucleic acid as well as quantification of each of the determined sequences and is summarized as follows.

Using, as a template, a target nucleic acid such as a single-stranded DNA or RNA containing a sequence of interest that specifies the identity of a nucleic acid in a sample, along with a primer containing a sequence specific to a downstream region of the sequence of interest in the target nucleic acid sequence and a random sequence and a known adapter sequence both located upstream to the specific sequence, a single stranded nucleic acid complementary to the target nucleic acid is synthesized by a DNA synthetase. The synthesized single-stranded nucleic acid thus contains, in order from upstream, the adapter sequence, the random sequence, and a sequence complementary to the sequence of interest. After the synthesis of the single-stranded nucleic acid, the excess primers are digested and removed by an enzyme specific to single-stranded nucleic acid such as exonuclease I.

Next, using a primer that specifically binds to the adapter sequence and a primer having a sequence specific to an upstream region of the sequence of interest in the target nucleic acid sequence, PCR is performed to amplify a nucleic acid fragment containing the random sequence and the sequence of interest. The amplified PCR products thus contain at least the random sequence and the sequence of interest. The sequences of the PCR products are then comprehensively determined by using a next-generation sequencer using, for example, MiSeq available from Illumina, Inc. Since the relationship between the number of different sequences of the random sequence present at one terminal of the determined sequence and the number of the sequences of interest (or the target nucleic acid containing the sequence of interest) in a sample follows the Poisson distribution, the number of the target nucleic acids containing the sequence of interest in a sample can be estimated from the number of different sequences of the random sequence.

According to conventional approaches, two or more target nucleic acids present in a sample are subjected to PCR and subsequently sequenced to obtain a sequence library. However, the ratio that compares the numbers of the respective target nucleic acid sequences in this sequence library does not reflect the ratio of the numbers of the target nucleic acids in the sample. This is due to the lack of quantitativity of PCR. For this reason, separate quantitative assays need to be performed for quantifying the respective sequences. This makes the conventional approaches inapplicable to comprehensive quantification of several hundred to several thousand different species of microorganisms that are detected, for example, in a 1 ml sample of oceanic sediments.

In contrast, the quantification method of one embodiment of the present invention permits sequencing of multiple nucleic acids while simultaneously enabling high-precision, comprehensive quantification of respective sequences.

Specifically, according to the quantification method of one embodiment of the present invention, the number of different sequences (i.e., variety) of the random sequence incorporated into the resulting amplicon is determined only by the synthesis of the single-stranded nucleic acid in the first step. The subsequent 20 to 40 cycle PCR may produce varied amounts of PCR products since the PCR efficiency can vary from one sequence to another. However, the PCR does not affect the variety of the incorporated random sequences.

Since the variety of the random sequence is determined statistically, sequencing sufficient amounts of the sequence using a next-generation sequencer allows the calculation of the copy numbers of target nucleic acid used as the initial template.

This is demonstrated by Examples described below. For example, according to the quantification method of one embodiment of the present invention, the calculated copy numbers are 70 to 80 % relative to the total copy numbers of two target nucleic acids that are introduced. This is highly accurate even considering the operational variation and other factors. In addition, the ratio of the two target nucleic acids is accurately reproduced independently of the mixing proportions or sequence specificity. Given such results, the quantification method of one embodiment of the present invention is likely to have a significant impact not only in academic fields such as ecology and biology, but also in various medical fields.

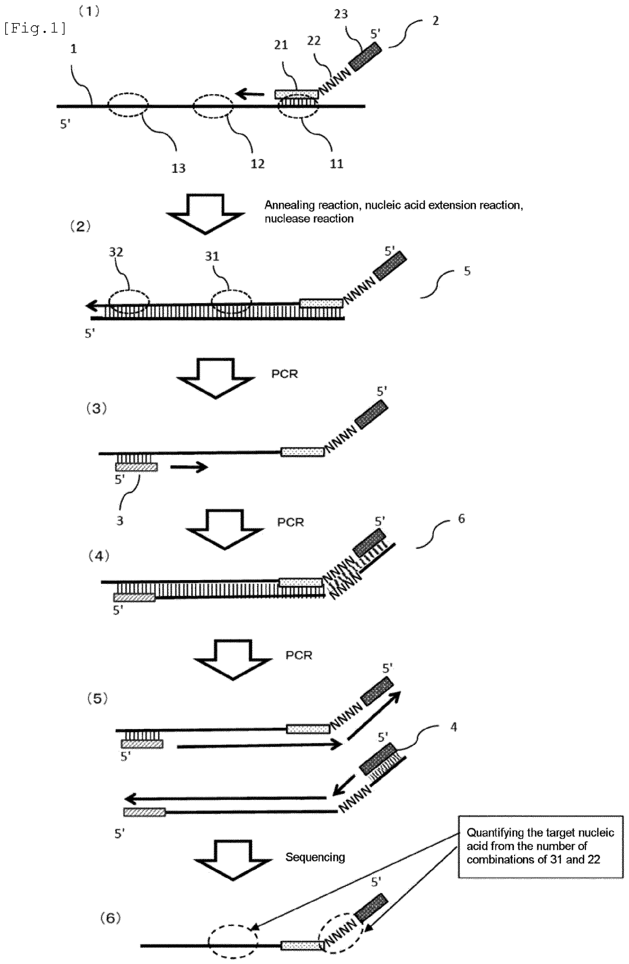

The quantification method of one embodiment of the present invention will now be described with reference to FIG. 1, which is a non-limiting and schematic representation of the quantifying method.

As shown in (1) in FIG. 1, a target nucleic acid 1, which is a single-stranded nucleic acid and contains, from its 3' end to 5' end, a first sequence 11, a sequence of interest 12 and a second sequence 13, is used as a template. When the target nucleic acid is a double-stranded nucleic acid, the double-stranded nucleic acid is subject to denaturation reaction to obtain a single-stranded nucleic acid to serve as the target nucleic acid 1.

A first primer 2, which contains, from its 5' end to 3' end, a specific sequence 23, a random sequence 22 and a sequence 21 complementary to the first sequence, is used on the target nucleic acid 1. The first primer 2 comprises primers having a number of different sequences corresponding to the number of different sequences of the random sequence 22. Specifically, when there are 1000 different sequences of the random sequence 22, there will be 1000 different sequences of the first primer 2.

The target nucleic acid 1 and the first primer 2 are annealed to allow the sequence 21 of the first primer 2 to hybridize to the first sequence 11 of the target nucleic acid 1. The hybrid of the target nucleic acid 1 and the first primer 2 is then subjected to nucleic acid extension using the target nucleic acid 1 as a template to cause the extension of nucleic acid from the sequence 21 of the first primer 2 in the direction of 5' to 3' end. The resulting extension products are then treated with nucleases to digest any unhybridized excess first primers 2, the single-strand portion of the target nucleic acid 1 and the other single strand that was not used as the template if the target nucleic acid was double-stranded. As a result, a first double-stranded nucleic acid 5 is obtained with one strand containing in direction from 5' end toward 3' end the specific sequence 23, the random sequence 22, the sequence 21 complementary to the first sequence, a sequence 31 complementary to the sequence of interest and a sequence 32 complementary to the second sequence (See, (2) in FIG. 1).

Subsequently, using the first double-stranded nucleic acid 5 as a template along with a second primer 3 containing the second sequence 13 and a third primer 4 containing the specific sequence 23, PCR is performed to obtain a second double-stranded nucleic acid 6 (See, (3) to (5) in FIG. 1). In this PCR, one strand of the first double-stranded nucleic acid 5 that contains, from its 5' end to 3' end, the specific sequence 23, the random sequence 22, the sequence 21 complementary to the first sequence, the sequence 31 complementary to the sequence of interest and the sequence 32 complementary to the second sequence is used as a template so as to carry out nucleic acid extension along with the second primer 3 (See, (3) in FIG. 1). As a result, a second double-stranded nucleic acid 6 is obtained (See, (4) in FIG. 1). Subsequently, using the second double-stranded nucleic acid 6 as a template along with the second primer 3 and the third primer 4 (See, (5) in FIG. 1), the reaction proceeds to produce the amplicon of the second double-stranded nucleic acid 6. The resulting second double-stranded nucleic acid 6 comprises a number of different sequences corresponding to the number of different sequences of the random sequence 22.

Subsequently, the sequence of the resulting second double-stranded nucleic acid 6 is determined and based on the determined sequence, the number of combinations of the sequence of interest 12 and the random sequence 22 is measured. The result is used in the above-described equation (1) to quantify the target nucleic acid (See, (6) in FIG. 1).

The target nucleic acid is not particularly limited: examples include those commonly known as nucleic acids, such as DNA and RNA. Preferably, the target nucleic acid is a nucleic acid derived from one or two or more organisms. More preferably, the target nucleic acid is a nucleic acid derived from one or two or more bacteria, archaea, viruses, eukaryotes, protozoa, plants, animals or insects.

Preferably, the target nucleic acid contains a variable region that varies among species and conserved regions that are common or well-conserved among species and located downstream and upstream of the variable region. In the present case, the variable region corresponds to the sequence of interest, the conserved region downstream (3' end side) of the variable region corresponds to the first region, and the conserved region upstream (5' end side) of the variable region corresponds to the second region. The nucleic acid that contains a variable region and conserved regions downstream and upstream of the variable region is not particularly limited: examples include nucleic acids containing part of all of a gene, such as rRNA gene and other functional genes.

The functional gene is not particularly limited. PCR-based sequencing and quantification of functional genes is well executed as common general technical knowledge in the art. For example, target sequences including a functional gene of each species and its upstream and downstream sequences is searched on an existing database. The sequences can be then aligned to find common sequences (i. e., conserved regions) that can serve as the target for primers used in PCR. Since most functional genes include conserved regions, such techniques may be used to obtain unknown sequences of functional genes (with known functions) from environmental DNA. For example, in the case of eukaryotes, whose genes contain a large proportion of non-gene regions, the upstream and downstream common sequences are often used as primers. Other techniques include the techniques disclosed in http://togodb.biosciencedbc.jp/entry/stga_howto/5

As used herein, the term "conserved region" is not limited to conserved regions in contrast with variable regions in rRNA gene; rather it is intended to mean any region containing a sequence common among each species.

Specific examples of target nucleic acid include rRNA genes. For the 16S rRNA gene found in bacteria and archaea, a nucleic acid containing any of the V1 through V9 regions that are variable regions of the 16S rRNA gene (sequence of interest) and the conserved regions downstream and upstream of the variable regions (first and second sequences) can serve as the target nucleic acid.

The number of bases in the first sequence, the sequence of interest and the second sequence of the target nucleic acid is not particularly limited: for example, the first sequence may include a sufficient number of bases to hybridize with the first primer while the second sequence may include a sufficient number of bases to act as the second primer. Preferably, the sequences include about 10 to about 50 bases.

The target nucleic acid may be pure nucleic acid or nucleic acid present in a sample. For example, the target nucleic acid may be nucleic acid present in water, soil, air, tissue of a living body, food products, pharmaceutical products, cosmetic products, or the like. When the target nucleic acid is a nucleic acid present in a sample, it is preferred to extract the nucleic acid from the sample in advance in consideration of common technical practices in the art.

The target nucleic acid may be present in any number that allows the quantification of the target nucleic acid. For example, the number of the target nucleic acid present may be in a range of 100 to 1,000,000 in terms of the sensitivity and the lower and upper limits of detection of the quantification method of one embodiment of the present invention.

The random sequence may be any sequence designed to have a random base sequence. When the random sequence comprises adenine (A), thymine (T), cytosine (C) and guanine (G), the number of the bases in the random sequence is preferably any number in a range of 4 to 20 in terms of the type, number and readiness of design of the target nucleic acid to be quantified. Similarly, the number of different sequences (type) of the random sequence is preferably in a range of $10^2$ to $10^{15}$.

The sequence and the number of bases of the specific sequence are not particularly limited. In terms of the function of the third primer containing the specific sequence as a primer as well as the readiness in designing such sequences, the number of bases in the specific sequence is preferably any number between 10 and 50. More preferably, the specific sequence is comprised of a sequence that is non-complementary to the sequence of the target nucleic acid.

The sequence complementary to the first sequence may be any sequence having a sufficient number of bases to specifically hybridize with the first sequence.

In the step (1) of the quantification method of one embodiment of the present invention, annealing, nucleic acid extension and nuclease reactions are carried out using the target nucleic acid as the template along with a number of first primers of different sequences with the number of different sequences of the first primers corresponding to the number of different sequences of the random sequence. The number of different sequences of the first primer may be the same as, greater than, or less than the number of different sequences of the random sequence.

In the step (1) of the quantification method of one embodiment of the present invention, the annealing, the nucleic acid extension and the nuclease reactions may be carried out using any of the techniques commonly used by those skilled in the art. While the annealing reaction in the step (1) of the quantification method of one embodiment of the present invention is not particularly limited, it may refer to a reaction in which the first primer and the target nucleic acid are placed under conditions such that the first primer hybridizes with the target nucleic acid, either with or without a preceding thermal reaction (denaturation). If the annealing reaction is preceded by denaturation (e. g., a reaction in which the first primer and the target nucleic acid are heated to 90°C to 100°C for several tens of seconds to several minutes), then the annealing reaction may be able to be carried out by rapidly cooling and maintaining the reaction system at about 50°C to about 65°C for several tens of seconds to several minutes. If the annealing reaction is not preceded by denaturation, then the annealing reaction may be able to be carried out by maintaining the reaction system at about 30°C to about 65°C for several tens of seconds to several minutes.

The nucleic acid extension reaction is not particularly limited: for example, it may refer to any reaction that uses DNA polymerases and other nucleic acid synthetases and that is carried out at temperatures suitable for the enzymatic activity of the nucleic acid synthetases. The nuclease reaction is not particularly limited: for example, it may refer to any reaction that uses nucleases such as exonucleases and endonucleases and that is carried out at temperatures suitable for the nucleases. After the nuclease reaction, it is preferred to deactivate the nucleases, such as by heating, so that the nucleases will not affect the subsequent PCR.

By carrying out the step (1) of the quantification method of one embodiment of the present invention, a first double-stranded nucleic acid is obtained with one strand containing in direction from 5' end toward 3' end the specific sequence, the random sequence, the sequence complementary to the first sequence, a sequence complementary to the sequence of interest, and a sequence complementary to the second sequence.

In the step (2) of the quantification method of one embodiment of the present invention, PCR is carried out using the first double-stranded nucleic acid obtained from the step (1) as a template along with a second primer containing the second sequence and a third primer containing the specific sequence. This gives a second double-stranded nucleic acid.

The second primer may be any sequence that contains the second sequence. For example, the second primer may be the second sequence itself or may contain other sequences in addition to the second sequence. Other sequences may include a random sequence. The second sequence in the second primer may be any base length as long as the second primer can achieve the desired primer function of specifically binding to the sequence complementary to the second sequence.

The third primer may be any sequence that contains the specific sequence. For example, the third primer may be the specific sequence itself or may contain other sequences in addition to the specific sequence. The specific sequence in the third primer may be any base length as long as the third primer can achieve the desired primer function of specifically binding to the sequence complementary to the specific sequence.

Conditions for PCR are not particularly limited. Specifically, in view of the state of the art, appropriate temperatures and reaction time are selected so that denaturation, which separates a double-stranded nucleic acid into single strands, annealing, and nucleic acid extension by nucleic acid synthetases such as DNA polymerase can occur. PCR can then be performed by repeating these reactions for several tens of cycles. The nucleic acid synthetase used in PCR may be the same enzyme used in the nucleic acid extension reaction during the step (1) or different enzymes may be used.

The second double-stranded nucleic acid obtained from the PCR performed in the step (2) is a double-stranded nucleic acid with one strand containing, from its 5' end to 3' end, the specific sequence, the random sequence, the sequence complementary to the first sequence, the sequence complementary to the sequence of interest, and the sequence complementary to the second sequence and the other strand containing, from its 5' end to 3' end, the second sequence, the sequence of interest, the first sequence, a sequence complementary to the random sequence, and a sequence complementary to the specific sequence.

In the step (3) of the quantification method of one embodiment of the present invention, the sequence of the second double-stranded nucleic acid obtained from the step (2) is determined. The technique used to determine the second double-stranded nucleic acid is not particularly limited: for example, sequencing technologies based on the state of the art may be used. The sequencing technology applied may be any next-generation sequencing technology that enables simultaneous determination of multiple sequences. One specific sequencing technologies include a technique by which indexed DNA sequences to which index sequences have been added for sequencing are sequenced by MiSeq DNA sequencer (Illumina Inc.) as described in the following Examples. One strand, the other strand, or both strands of the second double-stranded nucleic acid may be sequenced.

In the step (4) of the quantification method of one embodiment of the present invention, the number of combinations of the sequence of interest and the random sequence is measured based on the sequence determined in the step (3) to quantify the target nucleic acid by the above-described equation (1).

The technique used to measure the number of combinations of the sequence of interest and the random sequence is not particularly limited: for example, the number can be calculated by allocating the number of different sequences of the random sequence for each sequence derived from each organism of interest based on the determined sequence information.

The number of the target nucleic acids can be calculated by the above-described equation (1) using the number of the combinations of the sequence of interest and the random sequence and the number of the random sequences.

In the quantification method of one embodiment of the present invention, various other steps or manipulations may be added before, after, or during each of the above-described steps as long as the objectives of the present invention can be achieved. For example, after the step (3) and before the step (4), the second double-stranded nucleic acid may be purified by a common DNA purification technique. In particular, the PCR product obtained from the step (3) may be subjected to agarose gel electrophoresis and the desired band may be excised.

Also disclosed is a method for amplifying a double-stranded nucleic acid containing a sequence of interest of a target nucleic acid and a random sequence, the method comprising at least the following steps (1) and (2):

(1) carrying out an annealing reaction, a nucleic acid extension reaction, and a nuclease reaction by using as a template a single-stranded nucleic acid of target nucleic acid or a single-stranded nucleic acid obtained from a double-stranded nucleic acid of target nucleic acid that contains in direction from 3' end toward 5' end a first sequence,

a sequence of interest and a second sequence, along with a number of first primers that each contain in direction from 5' end toward 3' end a specific sequence, a random sequence and a sequence complementary to the first sequence and that each have a different sequence corresponding to the number of different sequences of the random sequence so as to obtain a first double-stranded nucleic acid with one strand containing in direction from 5' end toward 3' end the specific sequence, the random sequence, the sequence complementary to the first sequence, a sequence complementary to the sequence of interest and a sequence complementary to the second sequence; and (2) carrying out a PCR using the first double-stranded nucleic acid obtained from the step (1) as a template along with a second primer containing the second sequence and a third primer containing the specific sequence so as to obtain a second double-stranded nucleic acid.

The steps (1) and (2) of the amplification method can be carried out with reference to the steps (1) and (2).

Also disclosed is a kit for quantifying a target nucleic acid that is either a single-stranded nucleic acid containing in direction from 3' end toward 5' end a first sequence, a sequence of interest and a second sequence or a double-stranded nucleic acid including the single strand, the kit comprising at least the following components (1) to (4):

(1) a number of first primers that each contain in direction from 5' end toward 3' end a specific sequence, a random sequence and a sequence complementary to the first sequence and that each have a different sequence corresponding to the number of different sequences of the random sequence;
(2) a nuclease;
(3) a second primer containing the second sequence; and
(4) a third primer containing the specific sequence.

The first, second and third primers may be obtained from any source as long as they contain each prescribed sequence. For example, the primers may be synthesized by modifying a commercially available primer containing the sequence complementary to the first sequence to sequentially add the random sequence and the specific sequence to the 5' end-side.

The number of different sequences of the first primer may be larger or smaller than, or the same as, the number of different sequences of the random sequence. The second primer may each comprise one sequence or more than one different sequences as long as the second primer contains the second sequence while the third primer may each comprise one sequence or more than one different sequences as long as the third primer contains e the specific sequence.

The nuclease may be any nuclease that has the activity to digest single-stranded nucleic acids. For example, it may be one or both of endonuclease and exonuclease. The nuclease may be obtained from any source. For example, commercially available nucleases may be used.

In addition to the above-described components (1) to (4), the kit may include other components such as buffers and nucleic acid synthetases. The kit can be used to perform the quantification method and the amplification method.

The present invention will now be described more specifically with reference to the following Examples, which are not intended to limit the present invention.

Examples

[Example 1. Evaluation of abundance of microorganisms using two microorganisms]

1. Target DNA

Genomic DNAs of *Methanocaldococcus jannaschii* and *Streptomyces avermitilis* were used as target DNA (Both provided by RIKEN BRC). The two genomic DNAs were mixed together at ratios shown in Table 1 and the resulting mixtures were used as sample DNAs. Each sample DNA was prepared so that the concentration of DNA is $10^4$ copies/$\mu$l.

[Table 1]

| Sample No. | *M. jannaschii* | *S. avermitilis* |
|:---:|:---:|:---:|
| 1 | 1 | 100 |
| 2 | 1 | 10 |
| 3 | 1 | 1 |

2. Primers

The primers used were 515F_mod primer (See, SEQ ID NO: 1 in Table 2) and 806R_mod primer (See, SEQ ID NO: 2 in Table 2) that were obtained through modification of 515F primer and 806R primer, respectively. The 515F and 806R primers are primers commonly used to amplify V4 region of 16S rRNA gene of bacteria and archaea. In Table 2, the underlined sequences represent adapter sequences used for a sequencer available from Illumina Inc.; N represents A, T, G or C; and lower case sequences represent sequences that bind to the template DNAs. Primer-F primer having a partial sequence of 515F_mod primer was also used (See, SEQ ID NO: 3 in Table 2). 65,536 different primers were used for each of 515F_mod primer and 806R_mod primer so as to correspond to the number of different sequences (65,536) of the random sequence (NNNNNNNN).

[Table 2]

| Name | Sequence (5'-3') |
|------|------------------|
| 515F_mod (SEQ ID: 1) | <u>TCGTCGGCAGCGTCAGATGTGTATAAGAGACAG</u>NNNNNNNNtgycagc mgccgcggtaa |
| 806R_mod (SEQ ID: 2) | <u>GTCTCGTGGGCTCGGAGATGTGTATAAGAGACAG</u>NNNNNNNNggact achvgggtwtctaat |
| Primer- F (SEQ ID: 3) | TCGTCGGCAGCGTCAGAT |

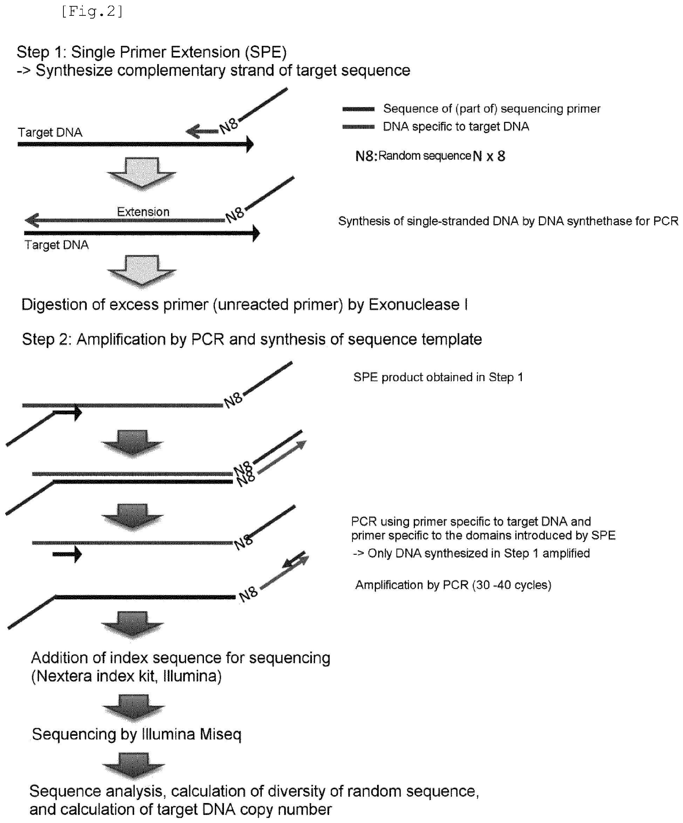

3. Single Primer Extension (SPE); See, Procedure 1 in FIG. 2

First, using 515F_mod primer (SEQ ID NO: 1 in Table 2), the complementary strand of 16S rRNA gene was synthesized. Specifically, reaction mixtures for single primer extension having respective compositions shown in Table 3 and containing respective sample DNAs were prepared and the mixtures were subjected to a single cycle of 98°C for 2 min, 55°C for 30 sec and then 68°C for 10 min to produce SPE products. Following the reaction, 1 μl of 10U to 20U exonuclease I (Takara Bio Inc.) was added to each mixture so as to become a total volume of 21 μl and the mixture was incubated at 37°C for 120 min to digest unreacted excess primer. Subsequently, the reaction mixture was incubated at 80°C for 30 min to deactivate exonuclease I.

[Table 3]

| Component | Volume (μl) |
|-----------|-------------|
| 2 x MightyAmp buffer ( Takara Bio Inc.) | 10.0 |
| 515F_mod (10 μM) | 0.6 |
| Sample DNA | 2.0 |
| Mighty Amp DNA polymerase ( Takara Bio Inc.) | 0.4 |
| Water | 7.0 |
| Total | 20.0 |

4. PCR and synthesis of sequencing template (See, Procedure 2 in FIG. 2)

Using, as the template, the SPE product obtained in the single primer extension described in 3 above, along with 806R_mod primer and Primer-F primer, PCR was performed. Specifically, a PCR reaction mixture shown in Table 4 containing 5 μl of each of the SPE products was prepared and PCR was performed by initial denaturation at 98°C for 2 min, followed by 40 cycles of 98°C for 10 sec, 55°C for 15 sec and then 68°C for 30 sec. The resulting PCR product was subjected to agarose gel electrophoresis and the desired band was excised to purify the DNA. Using Nextera Index Kit (Illumina Inc.,), an index sequence for sequencing was added to the purified DNA. The indexed DNA sequence was then sequenced on MiSeq DNA sequencer (Illumina Inc.).

[Table 4]

| Component | Volume (μl) |
|-----------|-------------|
| 2 x MightyAmp buffer ( Takara Bio Inc. ) | 10.0 |

(continued)

| Component | Volume ($\mu$l) |
|---|---|
| Primer-F (10 $\mu$M) | 0.6 |
| 806R_mod (10 $\mu$M) | 0.6 |
| SPE Product | 5.0 |
| Mighty Amp DNA polymerase ( Takara Bio Inc. ) | 0.4 |
| Water | 4.0 |
| Total | 20.0 |

5. Calculation of copy number of target DNA

The data for the determined sequences was processed with Mothur (http://www.mothur.org) to classify the sequences into those for *M. jannaschii* and those for *S. avermitilis.* The number of different sequences for the 8-base-long random sequence attached to the most upstream of the 16S rRNA sequence was counted. If the number of different sequences of the introduced random sequence (In the present example, $4^8$ = 65,536 different sequences) is sufficiently large relative to the copy number of the target DNA, it can be assumed that the number of different sequences of the random sequence follows the Poisson distribution. Based on this assumption, the copy number of the target DNA was determined from the number of different sequences of the random sequence using the following equation:

$$N = ln\left(\frac{C - H}{C}\right) \times (-C)$$

where N = copy number of target DNA sequence, C = number of different sequences of label (In this study, 65,536), and H = counted number of different sequences of the label.

6. Evaluation

The results of the calculated copy numbers of target DNA are summarized in Table 5. The copy number of the target DNA sequence introduced in the sequencing reaction was 4,761 copies whereas the copy numbers obtained by the experiment were 3,333 to 3,923 copies, which were slightly less. However, since the expected value is the amount of DNA calculated from the OD value measured by an absorption spectrophotometer, it may differ from the amount of DNA actually used in the reaction. Also, the ratios of M. jannaschii to S.avermitilis were approximately equal to the respective expected values, indicating that the present method can accurately quantify individual microorganisms in a complex microorganism system.

[Table 5]

| | Measured value | | | | Theoretical value | |
|---|---|---|---|---|---|---|
| Sample | MJ copies | SA copies | Total copies | MJ/SA | Total copies | MJ/SA |
| 1 | 60 | 3273 | 3333 | 0.018 | 4761 | 0.010 |
| 2 | 362 | 3923 | 3923 | 0.102 | 4761 | 0.100 |
| 3 | 1903 | 3879 | 3879 | 0.964 | 4761 | 1.000 |

MJ=*M.jannaschii*, SA=*S.avermitilis*

[Example 2. Evaluation of abundance of microorganisms in a complex system]

1. Target DNA

Genomic DNAs of *Methanocaldococcus jannaschii* and *Halomonas elongata* (both provided by RIKEN BRC) were used to compare the results of quantification based on the number of sequences in a library after sequencing and the results of quantification based on the number of different sequences of random sequence. The genomic DNAs of M. jannaschii and H. elongata were mixed together at ratios shown in Table 6 and the resulting mixtures were used as sample DNAs. Each sample DNA was prepared so as to show a DNA concentration of $10^4$ copies/$\mu$l.

To confirm the linearity of the quantified values, the genomic DNA of *Sulfolobus tokodaii* (provided by RIKEN BRC) was serially diluted and used as a sample DNA. Each sample DNA was prepared so as to show a DNA concentration of $10^4$ copies/$\mu$l.

[Table 6]

| Sample No. | *M. jannaschii* | *H. elongata* |
|---|---|---|
| 1 | 1 | 1 |
| 2 | 9 | 1 |

2. Environmental DNA

Using Power Max Soil DNA Isolation Kit (Mo Bio), DNA was extracted from the mud collected from the volcanic vent of Mt. Garan, an active volcano located in Yufu, Oita prefecture, Japan, and also from hot spring water risen near the volcanic vent. The DNA was designated as environmental DNA.

3. Single Primer Extension (SPE)

The single primer extension reaction was carried out in the same manner as in "3. Single Primer Extension" in Example 1.

4. PCR and synthesis of sequencing template

PCR and purification of DNA were carried out in the same manner as in "4. PCR and synthesis of sequencing template" in Example 1. Using the purified DNA and an index-addition reaction mixture having respective composition shown in Table 7, an index sequence was added to the purified DNA for sequencing. The reaction was performed by initial denaturation at 95°C for 30 sec, followed by 8 cycles of 95°C for 30 sec, 55°C for 30 sec and then 72°C for 30 sec. This was followed by extension at 72°C for 5 min. Subsequently, the indexed DNA sequence was sequenced on MiSeq DNA sequencer (Illumina Inc.).

[Table 7]

| Component | Volume ($\mu$l) |
|---|---|
| 2 x KAPA HiFi HotStart Ready mix (kapabiosystems) | 12.5 |
| Primers (1 $\mu$M) from Nextera Index Kit (Illumina) | 5.0 |
| PCR Product | 2.5 |
| Water | 5.0 |
| Total | 25.0 |

5. Calculation of copy number of target DNA

In the same manner as in "5. Calculation of copy number of target DNA" in Example 1, the obtained sequence data of the target DNA was processed with Mothur (http://www.mothur.org) and if necessary, the sequences were sorted by the strain of microorganism. The number of different sequences for the 8-base-long random sequence attached to the most upstream of the 16S rRNA sequence was counted and the copy number of target DNA was then calculated.

6. Evaluation of quantification accuracy based on the number of different sequences of the random sequence

The proportions of copy number of *M. jannaschii* and *H. elongata* determined from the copy numbers of the target DNAs calculated based on the number of different sequences of the random sequence as described above was compared to the proportions of the numbers of the sequences for the respective bacterial species in a sequencing library obtained by mixing the genomic DNAs of the two species *M. jannaschii* and *H. elongata* and conducting sequencing in an ordinary manner. To conduct the sequencing in an ordinary manner, a reaction mixture having a composition shown in Table 8 was prepared and PCR was performed by initial denaturation at 98°C for 2 min, followed by 30 cycles of 98°C for 10 sec, 55°C for 15 sec and then 68°C for 30 sec. The resulting PCR products were sequenced using MiSeq.

[Table 8]

| Component | Volume ($\mu$l) |
|---|---|
| 2 x MightyAmp buffer (Takara Bio Inc.) | 10.0 |
| 515F_mod (10 $\mu$M) | 0.6 |
| 806R_mod (10 $\mu$M) | 0.6 |
| Template DNA (10,000 copies/$\mu$l) | 0.75 |
| Mighty Amp DNA polymerase (Takara Bio Inc.) | 0.40 |
| Water | 7.65 |
| Total | 20.0 |

The results of the comparison are shown in Table 9. As can be seen from Table 9, the quantification based on the number of different sequences of the random sequence gave the proportions close to the theoretical values, whereas the proportion for *M. jannaschii* found in the sequence library tended to be smaller. Such a tendency seems to have resulted in part from the difference in GC content in the 16S rRNA gene region among the bacterial species used. Specifically, it can be inferred that the amplification efficiency of 16S rRNA gene of MJ, which has GC content of 65%, has decreased as compared to that of HE, which has GC content of 56%. In contrast, the random tag quantification enabled quantification independently of the sequence specificity of genes of interest. These results indicate that the quantification based on the number of different sequences of the random sequence enables accurate determination of abundance of microorganisms present in environments.

[Table 9]

| Species | Ratio in library | Quantificated random tag | Theoretical value |
|---|---|---|---|
| HE | 0.359 | 0.917 | 1.000 |
| MJ | 3.12 | 9.317 | 9.000 |

*MJ: *M. jannaschii,* HE: *H. elongata*

7. Evaluation of linearity of quantified values of random sequence

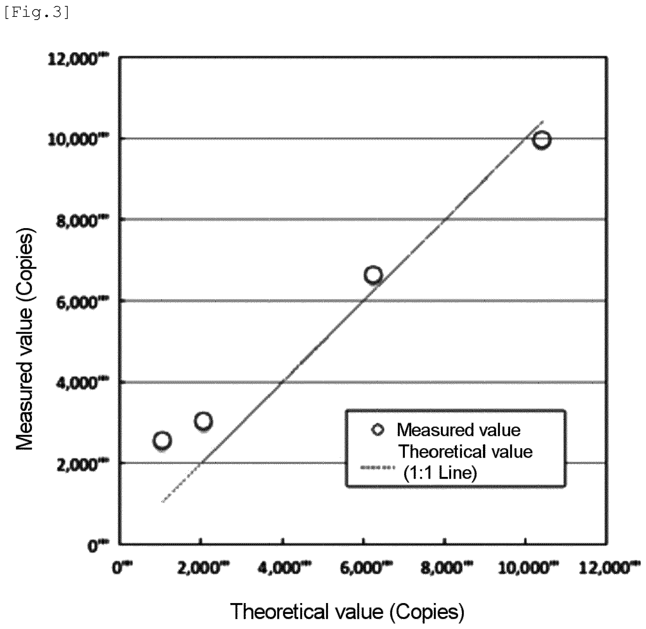

FIG. 3 shows the results of quantification performed using, as a template, the 16S rRNA gene of S. tokodaii at known copy numbers of $1.0 \times 10^3$, $2.0 \times 10^3$, $6.3 \times 10^3$, and $1.0 \times 10^4$.
While the measured value tended to be large at $1.0 \times 10^3$, the results showed good linearity at $2.0 \times 10^3$, $6.3 \times 10^3$, and $1.0 \times 10^4$ so that the measured values were close to the theoretical values. It can be inferred that increasing the label to 8 base-long or longer can lead to wider dynamic ranges.

8. Evaluation of application to complex system

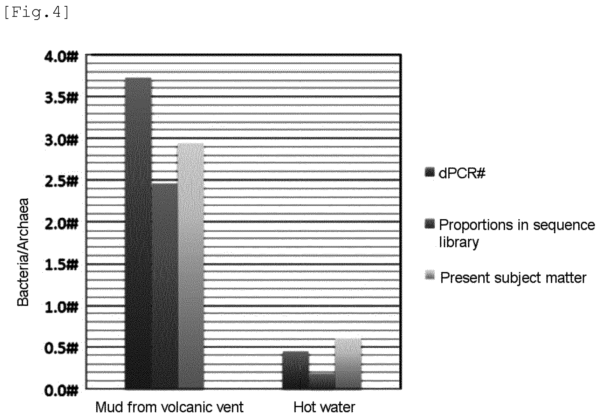

Quantification was performed on 16S rRNA gene present in natural environmental DNA. FIG. 4 shows comparison between the ratios of the numbers of bacteria/archaea in the sequencing library obtained by conducting sequencing in an ordinary manner and the ratios determined by the quantification based on the number of different sequences of the random sequence with the theoretical values being the copy numbers of 16S rRNA gene of bacteria and archaea obtained by digital PCR using Biomark HD (Fluidigm Corporation) in advance.
As shown in FIG. 4, the bacteria/archaea ratios determined by the quantification based on the number of different sequences of the random sequence tended to be closer to the bacteria/archaea ratios measured by digital PCR as compared to the bacteria/archaea ratios found in the sequencing library. These results suggest that the quantification based on the number of different sequences of the random sequence can accurately detect the abundance of microorganisms present in environments.
The sequences listed in the sequence listing are as follows:

[SEQ ID NO.1] 515F_mod primer tcgtcggcagcgtcagatgtgtataagagacagnnnnnnnnntgycagcmgccgcggtaa
[SEQ ID NO. 2]806R_mod primer gtctcgtgggctcggagatgtgtataagagacagnnnnnnnnnggactachvgggtwtctaat [SEQ ID

NO.3]Primer-F tcgtcggcagcgtcagat

Industrial Applicability

The method and the kit enable simple and accurate quantification of the copy number of target nucleic acids while enabling the sequencing of the target nucleic acids. Accordingly, the method and the kit may find applications in various technical fields requiring determination of the type, number and amount of organisms, including analysis of the type, number and amount of biological resources in various environments such as ocean, analysis of the type, number and amount of the bacterial flora in body tissue such as bowel, comprehensive and quantitative analysis of infectious and non-infectious viruses, and microbial limit tests for food products, pharmaceutical products and various other products.

Reference Numerals

1:    target nucleic acid
2:    first primer
3:    second primer
4:    third primer
5:    first double-stranded nucleic acid
6:    second double-stranded nucleic acid
11:   first sequence
12:   sequence of interest
13:   second sequence
21:   sequence complementary to first sequence
22:   random sequence
23:   specific sequence
31:   sequence complementary to sequence of interest
32:   sequence complementary to second sequence

**Claims**

1.  A method for quantifying a target nucleic acid, comprising the following steps (1) to (4):

    (1) carrying out

    - an annealing reaction,
    - a nucleic acid extension reaction, and
    - a nuclease reaction by using as a template a single-stranded nucleic acid of target nucleic acid or a single-stranded nucleic acid obtained from a double-stranded nucleic acid of target nucleic acid that contains in direction from 3' end toward 5' end
    - a first sequence,
    - a sequence of interest and
    - a second sequence,
    along with a number of first primers that each contain in direction from 5' end toward 3' end a specific sequence, a random sequence and a sequence complementary to the first sequence and that each have a different sequence corresponding to the number of different sequences of the random sequence,
    - so as to obtain a first double-stranded nucleic acid with one strand containing in direction from 5' end toward 3' end
    - the specific sequence,
    - the random sequence,

- the sequence complementary to the first sequence,
- a sequence complementary to the sequence of interest, and
- a sequence complementary to the second sequence;

(2) carrying out a PCR using the first double-stranded nucleic acid obtained from the step (1) as a template along with a second primer containing the second sequence and a third primer containing the specific sequence so as to obtain a second double-stranded nucleic acid;

(3) determining the sequence of the second double-stranded nucleic acid obtained from the step (2); and

(4) measuring the number of combinations of the sequence of interest and the random sequence based on the sequence determined in the step (3) and quantifying the target nucleic acid by the following equation (1):

$$ N = ln \left( \frac{C - H}{C} \right) \times (-C) $$

(Equation (1))

wherein N represents the number of target nucleic acids; C represents the number of different sequences of the random sequence; and H represents the measured number of combinations of the sequence of interest and the random sequence.

2. The method according to claim 1, wherein the target nucleic acid is a nucleic acid derived from one or two or more organisms.

3. The method according to claim 1, wherein the target nucleic acid is a nucleic acid containing a part or all of rRNA gene.

4. The method according to claim 1, wherein the sequence of interest is a variable region of rRNA gene and the first sequence and the second sequence are downstream and upstream conserved regions of the variable region, respectively.

5. The method according to claim 1, wherein the target nucleic acid is a nucleic acid in a sample selected from the group consisting of water, soil, air, body tissue, food products, pharmaceutical products and cosmetic products.

6. The method according to claim 1, wherein the number of the target nucleic acids is any number between 100 and 1,000,000.

7. The method according to claim 1, wherein the random sequence is a random sequence having any number of bases between 4 and 20 or the number of different sequences of the random sequence is any number between $10^2$ and $10^{15}$.

8. The method according to claim 1, wherein the specific sequence has any number of bases between 10 and 50 and comprises a sequence non-complementary to the sequence of the target nucleic acid.

**Patentansprüche**

1. Ein Verfahren zur Quantifizierung einer Zielnukleinsäure, umfassend die folgenden Schritte (1) bis (4):

(1) Durchführen von

- einer Anlagerungsreaktion,
- einer Nukleinsäure-Extensionsreaktion, und
- einer Nuklease-Reaktion unter Verwendung einer einzelsträngigen Nukleinsäure der Zielnukleinsäure als Matrize oder einer einzelsträngigen Nukleinsäure, die aus einer doppelsträngigen Nukleinsäure der Zielnukleinsäure erhalten wurde, als Matrize, wobei die einzelsträngige Nukleinsäure in Richtung vom 3'-Ende zum 5'-Ende folgendes enthält
- eine erste Sequenz,

- eine Sequenz von Interesse und
- eine zweite Sequenz,

zusammen mit einer Anzahl von ersten Primern, die jeweils in Richtung vom 5'-Ende zum 3'-Ende eine spezifische Sequenz, eine Zufallssequenz und eine zur ersten Sequenz komplementäre Sequenz enthalten und die jeweils eine unterschiedliche Sequenz entsprechend der Anzahl der unterschiedlichen Sequenzen der Zufallssequenz aufweisen,

- um eine erste doppelsträngige Nukleinsäure mit einem Strang zu erhalten, der in Richtung vom 5'-Ende zum 3'-Ende folgendes aufweist

- die spezifische Sequenz,
- die Zufallssequenz,
- die zur ersten Sequenz komplementäre Sequenz,
- eine Sequenz, die komplementär zur Sequenz von Interesse ist, und
- eine zur zweiten Sequenz komplementäre Sequenz;

(2) Durchführung einer PCR unter Verwendung der in Schritt (1) erhaltenen ersten doppelsträngigen Nukleinsäure als Matrize zusammen mit einem zweiten Primer, der die zweite Sequenz enthält, und einem dritten Primer, der die spezifische Sequenz enthält, um so eine zweite doppelsträngige Nukleinsäure zu erhalten;

(3) Bestimmen der Sequenz der zweiten doppelsträngigen Nukleinsäure, die aus dem Schritt (2) erhalten wurde; und

(4) Messen der Anzahl der Kombinationen aus der Sequenz von Interesse und der Zufallssequenz auf der Grundlage der in Schritt (3) bestimmten Sequenz und Quantifizierung der Zielnukleinsäure durch die folgende Gleichung (1):

$$N \;=\; ln \left( \frac{C - H}{C} \right) \times (-C)$$

(Gleichung (1))

worin N die Anzahl der Zielnukleinsäuren darstellt; C die Anzahl der verschiedenen Sequenzen der Zufallssequenz darstellt; und H die gemessene Anzahl von Kombinationen der Sequenz von Interesse und der Zufallssequenz darstellt.

**2.** Das Verfahren nach Anspruch 1, wobei die Zielnukleinsäure eine Nukleinsäure ist, die von einem oder zwei oder mehreren Organismen abgeleitet ist.

**3.** Das Verfahren nach Anspruch 1, wobei die Zielnukleinsäure eine Nukleinsäure ist, die einen Teil oder die Gesamtheit eines rRNA-Gens enthält.

**4.** Das Verfahren nach Anspruch 1, wobei die Sequenz von Interesse eine variable Region eines rRNA-Gens ist und die erste Sequenz und die zweite Sequenz jeweils in Leserichtung abwärts bzw. in Leserichtung aufwärts konservierte Regionen der variablen Region sind.

**5.** Das Verfahren nach Anspruch 1, wobei die Zielnukleinsäure eine Nukleinsäure in einer Probe ist, wobei die Probe ausgewählt ist aus der Gruppe bestehend aus Wasser, Boden, Luft, Körpergewebe, Lebensmittelprodukten, pharmazeutischen Produkten und kosmetischen Produkten.

**6.** Das Verfahren nach Anspruch 1, wobei die Anzahl der Zielnukleinsäuren irgendeine Zahl zwischen 100 und 1.000.000 ist.

**7.** Das Verfahren nach Anspruch 1, wobei die Zufallssequenz eine Zufallssequenz mit einer beliebigen Anzahl von Basen zwischen 4 und 20 ist oder die Anzahl der verschiedenen Sequenzen der Zufallssequenz eine beliebige Zahl zwischen $10^2$ und $10^{15}$ ist.

**8.** Das Verfahren nach Anspruch 1, wobei die spezifische Sequenz irgendeine Anzahl von Basen zwischen 10 und 50 aufweist und eine Sequenz umfasst, die zu der Sequenz der Zielnukleinsäure nicht komplementär ist.

**Revendications**

1. Procédé pour quantifier un acide nucléique cible, comprenant les étapes suivantes (1) à (4) consistant à :

   (1) effectuer

   - une réaction de renaturation,
   - une réaction d'extension d'acide nucléique et
   - une réaction de nucléase en utilisant comme matrice un acide nucléique simple brin d'un acide nucléique cible ou un acide nucléique simple brin obtenu d'un acide nucléique double brin d'un acide nucléique cible qui contient en direction d'une extrémité 3' vers une extrémité 5'
   - une première séquence,
   - une séquence d'intérêt et
   - une seconde séquence,
   avec un certain nombre de premières amorces qui contiennent chacune en direction de l'extrémité 5' vers l'extrémité 3' une séquence spécifique, une séquence aléatoire et une séquence complémentaire à la première séquence et que chacune a une séquence différente correspondant au nombre de séquences différentes de la séquence aléatoire,
   - de manière à obtenir un premier acide nucléique double brin avec un brin contenant en direction de l'extrémité 5' vers l'extrémité 3'
   - la séquence spécifique,
   - la séquence aléatoire,
   - la séquence complémentaire à la première séquence,
   - une séquence complémentaire à la séquence d'intérêt et
   - une séquence complémentaire à la seconde séquence ;

   (2) réaliser une PCR en utilisant le premier acide nucléique double brin obtenu à l'étape (1) comme une matrice avec une seconde amorce contenant la seconde séquence et une troisième amorce contenant la séquence spécifique de manière à obtenir un second acide nucléique double brin ;
   (3) déterminer la séquence du second acide nucléique double brin obtenu à l'étape (2) ; et
   (4) mesurer le nombre de combinaisons de la séquence d'intérêt et de la séquence aléatoire basé sur la séquence déterminée à l'étape (3) et quantifier l'acide nucléique cible par l'équation suivante (1) :

$$N = ln\left(\frac{C - H}{C}\right) \times (-C)$$

   (Équation (1))

   dans laquelle N représente le nombre d'acides nucléiques cibles ; C représente le nombre de séquences différentes de la séquence aléatoire ; et H représente le nombre mesuré de combinaisons de la séquence d'intérêt et de la séquence aléatoire.

2. Procédé selon la revendication 1, dans lequel l'acide nucléique cible est un acide nucléique dérivé d'un, de deux ou de plusieurs organismes.

3. Procédé selon la revendication 1, dans lequel l'acide nucléique cible est un acide nucléique contenant une partie du gène de l'ARNr ou tout le gène de l'ARNr.

4. Procédé selon la revendication 1, dans lequel la séquence d'intérêt est une région variable du gène de l'ARNr et la première séquence et la seconde séquence sont des régions conservées en aval et en amont de la région variable, respectivement.

5. Procédé selon la revendication 1, dans lequel l'acide nucléique cible est un acide nucléique dans un échantillon sélectionné parmi le groupe constitué de l'eau, du sol, de l'air, de tissus corporels, de produits alimentaires, de produits pharmaceutiques et de produits cosmétiques.

**6.** Procédé selon la revendication 1, dans lequel le nombre d'acides nucléiques cibles est tout nombre entre 100 et 1 000 000.

**7.** Procédé selon la revendication 1, dans lequel la séquence aléatoire est une séquence aléatoire ayant tout nombre de bases entre 4 et 20 ou le nombre de séquences différentes de la séquence aléatoire est tout nombre entre $10^2$ et $10^{15}$.

**8.** Procédé selon la revendication 1, dans lequel la séquence spécifique a tout nombre de bases entre 10 et 50 et comprend une séquence non complémentaire à la séquence de l'acide nucléique cible.

[Fig.1]

(1)

Annealing reaction, nucleic acid extension reaction, nuclease reaction

(2)

PCR

(3)

PCR

(4)

PCR

(5)

Sequencing

Quantifying the target nucleic acid from the number of combinations of 31 and 22

(6)

[Fig.2]

Step 1: Single Primer Extension (SPE)
-> Synthesize complementary strand of target sequence

Target DNA

Sequence of (part of) sequencing primer
DNA specific to target DNA

N8: Random sequence N x 8

Extension

Target DNA

Synthesis of single-stranded DNA by DNA synthethase for PCR

Digestion of excess primer (unreacted primer) by Exonuclease I

Step 2: Amplification by PCR and synthesis of sequence template

SPE product obtained in Step 1

PCR using primer specific to target DNA and
primer specific to the domains introduced by SPE
-> Only DNA synthesized in Step 1 amplified

Amplification by PCR (30 -40 cycles)

Addition of index sequence for sequencing
(Nextera index kit, Illumina)

Sequencing by Illumina Miseq

Sequence analysis, calculation of diversity of random sequence,
and calculation of target DNA copy number

[Fig.3]

[Fig.4]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110160078 A
- WO 2013128281 A1
- WO 2007129000 A2
- US 2015197786 A1

**Non-patent literature cited in the description**

- **GLENN K. FU et al.** *PNAS,* 31 May 2011, vol. 108 (22), 9026-9031

Sequence Listing

SEQUENCE LISTING

<110> JAPAN AGENCY FOR MARINE-EARTH SCIENCE AND TECHNOLOGY (JAMSTEC)

<120> A method for quantifying genes

<130> 16DF0511PCT

<160> 3

<170> PatentIn version 3.5

<210> 1
<211> 59
<212> DNA
<213> Artificial sequence

<220>
<223> Primer

<220>
<221> misc_feature
<222> (34)..(41)

<223> n is a, c, g, or t

<400> 1
tcgtcggcag cgtcagatgt gtataagaga cagnnnnnnn ntgycagcmg ccgcggtaa          59

<210> 2
<211> 62
<212> DNA
<213> Artificial sequence

<220>
<223> Primer

<220>
<221> misc_feature
<222> (35)..(42)
<223> n is a, c, g, or t

<400> 2

```
gtctcgtggg ctcggagatg tgtataagag acagnnnnnn nnggactach vgggtwtcta          60

at                                                                        62
```

<210> 3
<211> 18
<212> DNA
<213> Artificial sequence

<220>
<223> Primer

<400> 3
tcgtcggcag cgtcagat          18